# EUROPEAN PATENT APPLICATION

(11) **EP 2 568 041 A1**
(43) Date of publication of application: **13.03.2013**
(21) Application number: 12008214.4
(22) Date of filing: 19.12.2008
(51) Int. Cl.: C12N 9/64, C12N 9/96

(54) **Chemically modified factor IX**

(30) Priority: 27.12.2007 US 9263 P
(62) Divisional of application: 08868384.2
(71) Applicant: Baxter International Inc., Deerfield, IL 60015 (US); Baxter Healthcare S.A., 8152 Glattpark (Opfikon) (CH)
(72) Inventor: Turecek, Peter, 3400 Klosterneuburg (AT); Scheifflinger, Friedrich, 1090 Wien (AT)
(74) Representative: Müller-Boré & Partner Patentanwälte

(57) **Abstract**

The present invention discloses a chemically modified FIX, wherein the activation peptide region contains a covalently coupled water-soluble hydrophilic polymer.

## Description

### FIELD OF THE INVENTION

The present invention relates to a chemically modified blood coagulation factor IX (FIX) preparation.

### BACKGROUND OF THE INVENTION

Haemophilia B or Christmas disease, is a hereditary X-linked recessive bleeding disorder caused by a defect in clotting factor IX. Factor IX (FIX) is a single chain glycoprotein in plasma that plays an essential role in the intrinsic clotting pathway, where its activated form, factor IXa (FIXa), interacts with factor VIIIa, phospholipid and calcium ions to form the "tenase" complex which converts factor X to factor Xa.

FIX is synthesized as a single polypeptide chain 415 amino acids in length. FIX is present in blood as an inactive precursor molecule that consists of (1) a gamma-carboxyglutamic acid containing domain ("Gla domain"), (2) and (3) two epidermal growth factor-like domains ("EGF-1 domain", "EGF-2 domain"), (4) an activation peptide region ("AP region"), and (5) a serine protease domain. FIX undergoes extensive post-translational modification during transit through the endoplasmatic reticulum and Golgi apparatus: removal of the signal sequence; gamma-carboxylation of twelve Glu residues in the Gla domain by vitamin K dependent gamma-glutamyl carboxylase, a hepatic microsomal enzyme; N-glycosylation of N-157 and N-167 in the AP region; O-glycosylation of S-53 and S-61 in the Gla domain and T-159, T-169, T-172 and T-179 in the AP region; beta-hydroxylation at Asp-64 in the EGF-1 domain; sulfation of Tyr-155 and phosphorylation of Ser-158, both in the AP region.

In Haemophilia B, the deficiency is either in the amount or in the function of FIX. This disease is successfully treated by replacement therapy consisting of the administration of preparations of human plasma derived (pdFIX) or recombinant coagulation factor IX (rFIX). Plasma derived products are either prothrombin complex concentrates (which have been used in the past for the treatment of Haemophilia B) or purified FIX concentrates (mainly affinity purified factor IX). rFIX has been extensively characterised with respect to post-translational modifications. Despite minor differences to the pdFIX, specific activities and pharmacological effectiveness are comparable.

Biochemical comparison between pdFIX and CHO derived rFIX showed an indistinguishable secondary/tertiary structure as measured by fluorescence, circular dichroism or analytical ultracentrifugation. Minor differences were detected in post-translational modifications. Whereas in pdFIX all 12 Glu residues in the Gla domain are occupied (i.e. transformed to Gla), only 10 of the 12 sites are fully occupied in rFIX ("undercarboxylation" of Gla-40 or Gla-40 and Gla-36, respectively). N-linked glycans are fully sialylated and show high heterogeneity in pdFIX (however, this may also be due to the fact that pdFIX is prepared from plasma pools having diverse plasma donations); low hetereogeneity and often incomplete sialysation in rFIX. Ser-53 is Xyl-Xyl-Glc-glycosylated in rFIX whereas in pdFIX Ser-53 contains additional Xyl-Glc- glycosylation (Ser-61 contains NeuAc-Gal-GlcNAc-Fuc-in both forms). rFIX from CHO cells exhibits glycosylation with carbohydrates capped with sialic acid alpha(2-3)-galactose groups (CHO cells lack alpha(2-6)-sialyltransferase) whereas pdFIX contains terminal sialic acid alpha(2-6)-galactose moieties. Human host cells for expressing rFIX (such as HEK 293 cells) contain alpha(2-3)- and alpha(2-6)-sialyltransferases; accordingly HEK 293 derived rFIX differs in this respect from commercial CHO-derived rFIX (White et al., Thromb.Haemost. 78(1) (1997), 261-265; Bond et al., Sem.Hematol. 35 (2) (1998), Suppl.2, 11-17; Bebgie et al., Thromb.Haemost.94 (2005), 1138-1147).

It has been speculated whether a lower degree of phosphorylation of Ser-155 in the AP region and the lower degree of sulfation of Tyr-158 are responsible for the lower in-vivo recovery of rFIX (37.81 ± 14.0 % of rFIX compared to 52.61 ± 12.36 for pdFIX purified with monoclonal antibodies (White et al. (1997)). Griffith et al. (J.Thromb.Haemost. 5 (2007), Suppl.2: P-M-043) reported that N-Glycan sialylation is important for in vivo recovery of rFIX. In WO 2007/101681 A1 rFIX products with improved in vivo recovery are provided comprising at least 25 % and less than 98 % of fully phosphorylated and sulfated rFIX.

Elimination half life of CHO expressed rFIX and immunopurified pdFIX are comparable (18.10 ± 5.10 hours and 17.66 ± 5.31 hours, respectively (White et al., 1997)). Based on a report that deletion of the AP region (a del(155-177) mutant showed a terminal catabolic half life increase of 45 % compared to the wild-type form (Bebgie et al. (2005)), Chang et al. (J.Thromb.Haemost. 5 (2007), Suppl.2: O-M-088) treated FIX with neuraminidase and N- and O-glycanase to remove both, the N- and O-linked carbohydrates. De-glycosylated FIX had a significantly lower recovery than untreated FIX, whereas recovery of the de-glycosylated form were not statistically different in rFIX and pdFIX. It was therefore concluded that this suggested that glycosylation plays a major role in determining the recovery of FIX. It was further concluded that the role of sulfation/phosphorylation play a "relatively minor" role in in vivo recovery. Half life or activity data were not reported for the de-glycosylated forms of rFIX and pdFIX in Chang et al..

In clinical studies, rFIX has been shown to be safe and effective, but a 20 to 50 % higher dosage than for pdFLX is needed for successful treatment. This is due to a 30 to 50 % lower in vivo recovery for CHO derived rFIX than for pdFIX (as described above), as also revealed by pharmacokinetic data collected from pre-clinical and clinical studies, where pdFIX and rFIX are compared in different animal models, and clinical studies in haemophilia B patients. However, the circulating half-life of rFIX is not distinguishable from pdFIX preparations.

There had been various attempts to improve FIX drugs, e.g. (for rFIX) increased mRNA production, reduced binding to collagen IV, increasing the specific activity and improving the recovery by making rFIX more similar to pdFIX (Pipe, Sem.Thromb.Hemost. 30 (2) (2004), 227-237; WO 2007/101681 A1); (for pdFIX) enrichment and specific purification (US 5,639,857 A). However, there is still a strong need for improved FIX preparations which can be administered in a lower dosage or in larger time intervals than conventional FIX preparations for a successful treatment. Whereas most strategies in the prior art concentrate on improving recovery and increasing FIX activity, strategies which aim at prolongation of half life of the protein are rare, mainly because half life of rFIX and pdFIX are the same. This is mainly due to the known sensibility of the FIX protein against (even minor) chemical modification or mutations and the potential immunological effects of introducing mutations into a human protein (Bebgie et al. (2005); Kaufman, Thromb.Haemost. 79 (1998), 1068-1079; Hansson et al., J Thromb.Haemost. 3 (2005), 2633-2648; Wojcik et al., Biochem.J. 323 (1997), 629-636)

The present invention aims at providing FIX and pharmaceutical preparations containing FIX with improved half life of FIX.

### SUMMARY OF THE INVENTION

Therefore, the present invention provides an isolated FIX or a pharmaceutical preparation containing FIX wherein the FIX comprises a chemical modification in the activation peptide region. This chemical modification according to the present invention is the introduction of organic moieties in the AP region which increase the half life of FIX in the body of the subject to which the FIX is administered to. Accordingly, the present invention relates to FIX wherein the AP region comprises a covalently coupled water-soluble hydrophilic polymer, said polymer being absent from biologically produced FIX. For example, polyethylene glycol or carbohydrates are added to the AP region. The present invention also relates to a pharmaceutical composition comprising a purified rFIX preparation having improved half life according to the present invention for treating a bleeding disorder, e.g. haemophilia B. Furthermore, the present invention enables a method for treating a bleeding disorder comprising the step of administering a pharmaceutical composition comprising the FIX preparation with improved half life according to the present invention. In addition, the present invention also relates to a method for the production of a FIX wherein the AP region comprises a covalently coupled water-soluble hydrophilic polymer by covalently coupling the water-soluble polymer to FIX.

### DETAILED DESCRIPTION OF THE INVENTION

The present invention discloses a FIX, wherein the AP region comprises a covalently coupled water-soluble hydrophilic polymer. The polymer is e.g. chemically or enzymatically attached to a biologically produced FIX. Therefore, the water-soluble hydrophilic polymer according to the present invention is, of course, absent from a biologically produced form of FIX.

This strategy has surprisingly turned out to be applicable to FIX, despite the known lability that FIX usually shows with respect to chemical modification. It has been shown in the past by various reports that (chemically) affecting the detailed structure of the FIX molecules (especially with respect to its post-translational set-up) can be detrimental for its activity or at least leads to a significant reduction of its activity in vivo (Chang et al. (2007), WO 2007/101681 A, Atoda et al., J.Biol.Chem.281(14) (2006), 9314-9314; etc.). With the present invention, FIX is modified by covalent coupling with a water-soluble hydrophilic polymer which increases the half life of FIX in the circulation of a patient to whom the FIX is administered. Upon activation of the FIX according to the present invention, the AP region is excised (from Ala-146 to Arg-180) from FIX together with the polymer attached and FIXa is provided in vivo in its unmodified form, i.e. in the form without the covalently attached polymer (and the AP region). Therefore, the polymer is enzymatically released from FIX upon activation (together with the AP region). This is an elegant way of providing a "releasable" modification which serves in the circulation (for prolonging half life) as long as it is needed (i.e. immediately before the activity of FIX is needed as FIXa). It has been found out in the course of the present invention that the AP region is also chemically easily accessible and covalent coupling of the polymer to the AP region only (while leaving the rest of the FIX molecule unaffected (or selectively protected)) is manageable with the (bio-)chemistry available in the art.

"FIX" shall be any form of factor IX molecule with the typical characteristics of blood coagulation factor IX. FIX shall include FIX from plasma (pdFIX) and any form of rFIX which is capable of curing bleeding disorders in a patient which are caused by deficiencies in FIX (e.g. haemophilia B). FIX is comprised of the Gla domain, two EGF domains (EGF-1 and EGF-2), an AP region and a serine protease domain. FIX according to the present invention shall have the same amino acid sequence as human pdFIX and human rFIX and all functional variations thereof, i.e. variations (both, in amino acid sequence and post-translational modifications) which provide a comparable or improved in vivo activity of FIX. For curing the respective FIX related bleeding disorders in animals, the corresponding FIX sequences may be applied or those FIX forms which show sufficient cross-activity in related animal species. Furthermore, FIX according to the present invention shows all post-translational modifications necessary for a proper functioning of the protein in vivo. Ample literature is available describing functional forms of FIX, for example a naturally occurring Ala/Thr exchange at position 148; suitable FIX molecules which can be covalently coupled to the water-soluble hydrophilic polymers according to the present invention are described e.g. in White et al. (1997); Pipe (2004); WO 2007/101681 A1; US 5,639,857 A; Bebgie et al. (2005); Kaufman (1998); Hansson et al. (2005); Wojcik et al. (1997). Preferably, the FIX according to the present invention is a recombinantly produced FIX. The term "recombinant" when used with reference to FIX indicates that FIX has been produced by the introduction of a heterologous or non-naturally occurring nucleic acid or protein into a host cell, or the alteration of a native nucleic acid or protein in a host cell. Thus, for example, recombinant cells express genes that are not found within the native (non-recombinant) form of the cell, or express wild type and variant genes that are not in the native position in the genome of the cell, or express native genes that are otherwise abnormally expressed, under expressed or not expressed at all. The term "biologically produced" FIX covers all FIX forms being produced by organisms or cells without further chemical modification (not performable by such organisms or cells) after FIX has been isolated from such organisms or cells.

Commercially available recombinant factor IX products are often manufactured by using stable transfected Chinese hamster ovary (CHO) cells. CHO cells provide capacity for glycosylation and other post-translational modifications. With these cells, large-scale suspension cultures can be maintained without the addition of animal- or human-derived raw material. In the manufacture of one of these commercial products (marketed under the trade name Benefix(TM)) rFIX is co-expressed with the endopeptidase PACE/furin and is highly purified via multiple filtration and chromatographic steps.

The term "heterologous" when used with reference to portions of a nucleic acid indicates that.the nucleic acid comprises two or more subsequences that are not found in the same relationship to each other in nature. In one example, this term refers to a nucleic acid that is not in its native position in the genome. In another example, the nucleic acid is recombinantly produced, having two or more sequences from unrelated genes arranged to make a new functional nucleic acid, e.g. a promoter from one source and a coding region from another source. Similarly, a heterologous protein indicates that the protein comprises two or more subsequences that are not found in the same relationship to each other in nature (e.g. a fusion protein), or that it is a protein derived from a heterologous nucleic acid.

Any biologically active derivative of FIX may be modified by the present invention, thereby including any derivative of FIX having qualitatively the same functional and/or biological properties of FIX such as binding properties, and/or the same structural basis, such as a peptidic backbone. Minor deletions, additions and/or substitutions of amino acids of the polypeptide sequence of FIX which are not abolishing the biological activity of said polypeptide (i.e. reducing the activity to below 10 % or even below 5 % of the wild type form (= 100 %)) are also included in the present application as biologically active derivatives, especially those with improved specific activity (above 100 % activity of the wild-type form). The FIX according to the present invention may be derived from any vertebrate, e.g. a mammal. In one specific example of the present invention, the FIX is human FIX. The FIX according to the present invention may be produced by any method known in the art. This may include any method known in the art for the production of recombinant DNA by genetic engineering, e.g. via reverse transcription of RNA and/or amplification of DNA. Additionally, the recombinant DNA coding for FIX, e.g. a plasmid, may also contain a DNA sequence encoding a selectable marker for selecting the cells which have been successfully transfected with the plasmid. In an example of the present invention, the plasmid may also confer resistance to a selectable marker, e.g. to the antibiotic drug G418, by delivering a resistance gene, e.g. the neo resistance gene conferring resistance to G418.

The production of rFIX may include any method known in the art for the introduction of recombinant DNA into eukaryotic cells by transfection, e.g. via electroporation or microinjection. For example, the recombinant expression of human FIX can be achieved by introducing an expression plasmid containing the human FIX encoding DNA sequence under the control of one or more regulating sequences such as a strong promoter, into a suitable host cell line by an appropriate transfection method resulting in cells having the introduced sequences stably integrated into the genome. The calcium-phosphate co-precipitation method is an example of a transfection method which may be used according to the present invention.

The production of rFIX may also include any method known in the art for the cultivation of said transformed cells, e.g. in a continuous or batchwise manner, and the expression of the rFIX, e.g. constitutive or upon induction. For example, the nucleic acid coding for rFIX contained in the host organism of the present invention is expressed via an expression mode selected from the group consisting of induced, transient, and permanent expression. Any expression system known in the art or commercially available can be employed for the expression of a recombinant nucleic acid encoding rFIX, including the use of regulatory systems such as suitable, e.g. controllable, promoters, enhancers etc..

The production of rFIX may also include any method known in the art for the isolation of the protein, e.g. from the culture medium or by harvesting the transformed cells. For example, the rFIX-producing cells can be identified by isolating single-cell derived populations i.e. cell clones, via dilution after transfection and optionally via addition of a selective drug to the medium. After isolation the identified cell clones may be cultivated until confluency in order to enable the measurement of the rFIX content of the cell culture supernatant by enzyme-linked immunosorbent assay (ELISA) technique. Additionally, rFIX secreted by the cells may be identified for example by growing the cells in the absence of any growth promoting fetal bovine serum or components thereof. Vitamin K is added at appropriate concentrations to improve the functional properties of the rFIX protein. The supernatant may e.g. be harvested 24 hours after transfection. After identification, high rFIX producing cell clones may for example be further propagated and/or stored via cryopreservation. rFIX may be co-expressed with vitamin K reductase complex subunit 1 and/or furin.

Additionally, the production of FIX may include any method known in the art for the purification of FIX, e.g. via anion exchange chromatography or affinity chromatography. In one embodiment rFIX can be purified from cell culture supernatants by semi-affinity calcium-dependent anion exchange chromatography, e.g. in an endotoxin-free system. The purified FIX may be analyzed by methods known in the art for analyzing recombinant proteins, e.g. the ELISA technique, in addition, the protein integrity and activity may be assessed by measuring activated partial thromboplastin time (APTT) and by electrophoresis techniques including immunoblotting.

According to a preferred embodiment, the FIX according to the present is recombinant human FIX (rhFIX). The host cell type in which the rFIX according to the present invention is produced may be any mammalian cell with the ability to perform the proper posttranslational modifications of rFIX. For example a cell line selected from SkHep-, CHO-, HEK293-and BHK-cells. Many expression systems for FIX are thus available in the present field; however, FIX is preferably expressed in CHO- or HEK293-derived cells. Human cell lines such as HEK293 are preferred, if proper human sialyl residues are desired; however, also CHO derived FIX may be enzymatically engineered to provide an alpha(2-6)sialylation (Fischer et al., Thromb.Res.89 (1998), 147-150).

If it is desired to provide improved Tyr-155 sulfation and/or Ser-158 phosphorylation, FIX products having those properties are used for coupling the water-soluble polymers. Such FIX wherein Tyr-155 of FIX is sulfated and/or Ser-158 of FIX is phosphorylated are described e.g. in WO 2007/101681 A1.

The water-soluble hydrophilic polymer may be any polymer which is water-soluble and hydrophilic, i.e. a molecule that can transiently bond with water through hydrogen bonding. A further prerequisite of the polymer to be attached to FIX is of course that the polymer is pharmaceutically acceptable, i.e. that its administration to humans is not toxic, is causing no side effects or only side-effects which are tolerable in view of the patient's overall state (i.e. if the side effects are tolerable compared with the beneficial effects brought with the administration of the applied drug). This pharmaceutical acceptability should be present both, in the form covalently coupled to FIX and in the free or released form after degradation of the FIX conjugate or after the AP region (together with the polymer) has been excised from FIX in the course of activation of the molecule to FIXa. Finally, the polymer coupled to FIX should not elicit adverse immune responses. Accordingly, natural polymers to which the (human) body is adapted (and does not elicit an adverse immune response) or which are even based on human polymers are preferred.

Preferable polymers to be covalently coupled to the AP region of FIX are hydroxyethyl starch (HES), polyethylene glycol (PEG), dextran or polysialic acid (PSA).

HES is a nonionic starch derivative. It is frequently used as a blood plasma substitute and well accepted. Therefore, its use in connection with FIX is not critical. It can be prepared from waxy maize starch or potato starch. It may have a mean molecular weight of around 130 kDa. HES almost exclusively contains amylopectin and is therefore metabolized by plasma alpha-amylase in humans. In order to prevent or slow down such metabolization. the starch is modified by introduction of hydroxyethyl groups in the glucose units. Usually a molar substitution of around 0.4 is used when administered to humans which leads to a half life of HES to 1.4 hrs. However, HES can be provided in various forms with differing physico-chemical properties.

For example, US 5,502,043 A discloses an HES with a molecular weight MW of 110,000 to 150,000, a substitution level MS (molar substitution) of 0.38 to 0.5, preferably from 0.38 to 0.45, a substitution level DS (degree of substitution) of 0.32 to 0.45, preferably from 0.32 to 0.42, and a C2 /C6 ratio from 8 to 20, which showed a significant improvement of plasma viscosity and an improvement of microcirculation. Methods of arriving at these or other HES properties are described therein as well as e.g. in EP 0 402 724 A, GB 1,395,777, DE-A 28 14 032, DE-A 33 13 600 or WO 2005/082942 A.

HES can therefore easily be added to the AP region of FIX to carbohydrate moieties thereby leading to an increased half life of the modified FIX compared to FIX without the substitution with HES. Half life prolongation can be obtained with the knowledge already gathered with HES as a plasma volume expander as described above.

PEGylation of proteins is a well-established technique in protein chemistry and plays an important role in modem drug delivery. Many proteins or peptides are currently improved as therapeutic agents by using PEGylation. Improved PEGylated proteinaceous drugs such as Macugen, Neulasta, Pegasys or PEG-Intron have been successfully introduced in the market recently. PEGylation methods are well available in the present field, examples for protein PEGylation and detailed descriptions thereof are disclosed e.g. in Delgado et al. (Crit.Rev.Ther.Drug Carr.Syst. 9 (1992), 249-304); Fernandes et al. (Biochim.Biophys.Acta 1341 (1997), 26-34); Harris et al. (Clin. Pharmacokinet. 40 (7) (2001), 539-551); Bhadra et al. (Pharmazie 57(1) (2002), 5-29); Roberts et al. (Adv.Drug Del.Rev. 54 (2002), 459-476); DDT 10 (21) (2005) 1451-1458), etc..

To couple PEG to a protein it is necessary to activate the PEG by preparing a derivative of the PEG having a functional group at one or both termini. The functional group is chosen based on the type of available reactive group on the molecule that will be coupled to the PEG. For AP region of FIX, PEGylation is preferably provided at Ser- or Thr- residues. It is also possible to oxidise vicinal hydroxyl groups with periodate to form two reactive formyl moieties. In this case, all other carbohydrate moieties which should not be oxidised should be appropriately protected.

First-generation amine reactive PEG derivatives are e.g. PEG dichlorotriazine, PEG tresylate, PEG succinimidyl carbonate, PEG benzotriazole carbonate, PEG p-nitrophenyl carbonate, PEG trichlorophenyl carbonate, PEG carbonylimidazole and PEG succinimidyl succinate. Second generation PEG derivatives are e.g. mPEG-propionaldehyde, the acetal derivative of PEG-propionaldehyde or PEG-acetaldehyde, active esters of PEG carboxylic acids (obtainable e.g. by reacting the PEG-carboxylic acid with N-hydroxysuccinimide (NHS or HOSu) and a carbodiimide), PEG NHS esters based on propionic and butanoic acids and alpha-branched PEG-NHS esters based on propionic and butanoic acids. A specifically preferred form of PEGylation according to the present invention is the providing of releasable PEG groups, especially hydrolysable, PEGylation as described in chapter 3.2.4 of Roberts et al. (2002), Tsubery et al. (use of maleiimide-9-OH-methyl-7-sulfofluorene-N-OH-succinimide-OH-succinimide ester (MAL-FMS-OSU), a bifunctional agent which enables PEG chains to be linked to the AP region of FIX through a slowly hydrolysable chemical bond; J.Biol.Chem.279 (37) (2004), 38118-38124; WO 2004/089280 A).

PEGylation of the AP region of FIX is preferably carried out via Ser-158, Thr-159, Thr-163, Thr-169, Ser-171, Thr-172, Ser-174 or Thr-179, especially via Ser-158, Thr-163, Ser-171 or Ser-174, of FIX.

Dextran is a complex, branched polysaccharide made of glucose molecules joined into chains of varying lengths, used as an antithrombotic (anti-platelet), and to reduce blood viscosity. The straight chain consists of alpha(1,6) glycosidic linkages between glucose molecules, while branches begin from alpha(1,3) linkages (and in some cases, alpha(1,2) and alpha(1,4) linkages as well). Dextran is synthesized from sucrose by certain lactic-acid bacteria, for example Leuconostoc mesenteroides and Streptococcus mutans.

PSA is alpha-2,8-linked polysialic acid, a negatively charged hydrophilic polymer. It is a non-immunogenic biodegradable natural constituent of human tissue (on cell surfaces, in human milk; even expressed by bacteria to evade the immune system). PSA is hydrolysed in acidic environment (in vivo: lysosomal) e.g. at pH=5.5 and 37°C, to sialic acid. PSA can be coupled to the AP region of FIX e.g. by aldehyde coupling via a reductive amination step. First, vicinal OH-groups are oxidised in PSA (e.g. with NalO₄ or other suitable oxidation agents), then the oxidised PSA can be coupled to the AP region of FIX over a Schiff base and (e.g. after treatment with e.g. NaCNBH₃) stably connected to FIX by a secondary amine bond.

There are many ways of how to introduce the polymer according to the present invention to FIX. First, the AP region is an exposed region of FIX and therefore easily accessible. If other accessible regions of the FIX molecule are appropriately protected (e.g. by protecting groups or by linking or binding to molecules (such as physiological binding partners or specific antibodies) or by covering in tenside, lipid or membrane structures) the AP region can be covalently coupled to the polymer without affecting the rest of the molecule.

For example, various monoclonal antibodies (mAbs) exist for FIX with specific binding to various regions of FIV, such as the Gla domain, the EGF domains and the AP region. These antibodies have also been used for immunopurification of FIX. Binding to these antibodies is therefore reversible without significantly affecting the activity of FIX. Binding of the exposed regions to such antibodies (or to the natural binding partners) will protect these regions from the coupling chemistry according to the present invention. Alternatively, FIX can be bound to an mAb binding to the AP region, whereafter potentially reacting groups could be protected and - after release from the mAb - the coupling chemistry for the polymer can be applied to the (non-protected) AP region. The coupling can even be performed while. FIX is bound to a solid surface via such an mAb. This may be preferred for purification after the chemistry is performed on the FIX. In this case, the bound FIX to which the polymer has been coupled can be washed and selectively released from the solid surface in immunopurified quality.

Also selective enzyme activities or coupling chemistry can be used for specifically addressing the AP region. A preferred target of such selective coupling are the N-linked glycosylation groups of the AP region which are the only N-linked glycosyl residues in FIX. These targets can be specifically addressed, both enzymatically and via chemistry being selective for the N-glycosylation. It is therefore a preferred embodiment of the present invention that the water-soluble hydrophilic polymer is attached to FIX via Asn-157 and/or Asn-167 of FIX. The attachment of HES, PSA, PEG and dextran to these N-glycosylation sites (onto the carbohydrate structures) is therefore specifically preferred, especially for HES and PSA.

In an alternative embodiment, Tyr- and Ser-residues in the AP region of FIX according to the present invention may be covalently coupled to the polymer. This can be done after other potentially reactive groups (especially other Tyr- and Ser-residues of FIX) are appropriately protected from the coupling chemistry (see above). Suitable protection groups are any protection groups which are known in the field (especially other Tyr- and Ser-residues) and which do not irreversibly affect FIX activity. Accordingly, in an alternatively preferred embodiment, the water-soluble hydrophilic polymer is attached to FIX via Ser-158, Thr-159, Thr-163, Thr-169, Ser-171, Thr-172, Ser-174 or Thr-179, especially via Ser-158, Thr-163, Ser-171 or Ser-174, of FIX. This alternative is specifically preferred for PEGylation chemistry.

In a preferred embodiment, the water-soluble hydrophilic polymer is attached to FIX via a releasable linker, especially a hydrolysable linker. The chemistry for providing such hydrolysable linkers is available in the present field, examples are given e.g. in Roberts et al. (2002), Tsubery et al. (2004); WO 2004/089280 A (see above). Providing releasable linkers so that the polymer is released from FIX by e.g. hydrolysation or other release mechanisms (e.g. specific enzymatic activities) is advantageous, because a non-releasable polymer could result in a reduced activity, even in the AP region or at least make the proform FIX heavier and less exposed to activation in principle.

FIX according to the present invention will mainly be used for administering FIX activity to patients in need thereof, especially and primarily human patients. An important aspect of the present invention is therefore a pharmaceutical composition containing a FIX according to the present invention and a pharmaceutically acceptable carrier.

The pharmaceutical composition can be administered to a patient in need thereof and usually contains a dosage unit form to be applied to the patient. The pharmaceutically applicable composition according to the present invention therefore contains or comprises (if more than one administration amount is contained) a therapeutic dose or a therapeutically effective amount of FIX which is suitable to compensate or at least significantly alleviate the disorder to be treated. A "therapeutic dose" or "therapeutically effective amount" or "effective amount" of FIX or a composition comprising FIX is an amount of the FIX or composition comprising FIX which prevents, alleviates, abates, or reduces the severity of symptoms of bleeding disorders associated with functional defects of FIX or deficiencies of FIX. Frequency of administration of the FIX compositions described herein, as well as dosage, will vary from individual to individual, and may be readily established using standard techniques. Often 1, 2, 3, 4, or 5 doses are administered each week. In some cases, the doses are administered daily. In some cases, doses are administered 1, 2, 3, 4, or more times per day. Doses can also be administered on demand (e.g., following a trauma that causes bleeding in a subject or prior to a scheduled medical procedure expected to cause bleeding in a subject such as, for example surgery or dental work). A therapeutic dose is an amount of a compound that, when administered as described above, is capable of promoting an increased in vivo recovery of FIX e.g., Factor IX activity (e.g., as measured by APTT assays as set forth in, e.g., Chen et al. Adv Ther. 2003 Sep-Oct;20(5):231-6) following administration of the compositions to the individual. The compositions should also be capable of causing a response that leads to an improved clinical outcome (e.g., improved clotting time) in patients receiving the FIX as compared to patients who do not receive such treatment. Such responses may generally be evaluated using samples obtained from a patient before and after treatment. Suitable dose sizes will vary with the body weight of the patient, type of hemorrhage to be treated or prevented, and the desired plasma FIX concentration, but will typically range from about 10- 150, 20-100, 20-5-, or 40-50 International Units (IU) per kg body weight. An IU of FIX activity per kg body weight is typically equal to the FIX activity in 1 ml of fresh plasma and increases the FIX plasma concentration by 1%. Accordingly the composition according to the present invention contains FIX with a specific activity of at least 100 international units (IU) of FIX per mg FIX protein, especially at least 200 IU FIX per mg FIX protein.

The present pharmaceutical compositions may contain any suitable carrier known to those of ordinary skill in the art, including, for example, water, saline, alcohol, a fat, a wax, a buffer, a solid carrier, such as mannitol, lactose, starch, magnesium stearate, sodium saccharine, talcum, cellulose, glucose, sucrose, and magnesium carbonate. Suitable buffers include, e.g., neutral buffered saline or phosphate buffered saline. Additional suitable excipients include, e.g., carbohydrates (e.g., glucose, mannose, sucrose or dextrans), mannitol, proteins, polypeptides or amino acids such as histidine or glycine, antioxidants, bacteriostats, chelating agents such as EDTA or glutathione, detergents (e.g., fatty acid esters of sorbitan polyethoxylates such as, for example, polysorbate 20, polysorbate 60, or polysorbate 80), solutes that render the formulation isotonic, hypotonic or weakly hypertonic with the blood of a recipient, suspending agents, thickening agents and/or preservatives. Alternatively, compositions of the present invention may be formulated as a lyophilizate. The compositions described herein may be administered as part of a sustained release formulation (i.e. a formulation such as a capsule or sponge that effects a slow release of compound following administration). Such formulations may generally be prepared using well known technology (see, e.g., Coombes et al. (1996) Vaccine 14:1429-1438). Sustained- release formulations may contain a polypeptide, polynucleotide or antibody dispersed in a carrier matrix and/or contained within a reservoir surrounded by a rate controlling membrane. Carriers for use within such formulations are biocompatible, and may also be biodegradable; preferably the formulation provides a relatively constant level of active component release. Such carriers include microparticles of poly(lactide-co-glycolide), as well as polyacrylate, latex, starch, cellulose and dextran. Other delayed-release carriers include supramolecular biovectors, which comprise a non-liquid hydrophilic core (e.g., a cross-linked polysaccharide or oligosaccharide) and, optionally, an external layer comprising an amphiphilic compound (see, e.g., WO 94/20078; WO 94/23701; and WO 96/06638). The amount of active compound contained within a sustained release formulation depends upon the site of implantation, the rate and expected duration of release and the nature of the condition to be treated or prevented. As stated above, the pharmaceutical compositions according to the present invention may be presented in unit-dose or multi-dose containers, such as sealed ampoules or vials. Such containers are preferably hermetically sealed to preserve sterility of the formulation until use. In general, formulations may be stored as suspensions, solutions or emulsions in oily or aqueous vehicles. Alternatively, a pharmaceutical composition may be stored in a freeze-dried condition requiring only the addition of a sterile liquid carrier immediately prior to use.

It is advantageous to use similar pharmaceutical formulations as for the corresponding FIX preparations (without the polymers) or for the polymers themselves (if they have already been pharmaceutically administered). For example, BeneFIX® is sold with a specific activity of greater than or equal to 200 I.U. per milligram of protein; it contains no preservatives or added animal or human components. BeneFIX® is formulated as a sterile, nonpyrogenic, lyophilized powder preparation intended for intravenous (IV) injection. It is available in single use vials containing the labeled amount of factor IX activity, expressed in international units (I.U.). Each vial contains nominally 250, 500, or 1000 I.U. of Coagulation Factor IX (Recombinant). After reconstitution of the lyophilized drug product, the concentrations of excipients in the 500 and 1000 I.U. dosage strengths are 10 mM L-histidine, 1% sucrose, 260 mM glycine, 0.005% polysorbate 80. The concentrations after reconstitution in the 250 I.U. dosage strength are half those of the other two dosage strengths. The 500 and 1000 I.U. dosage strengths are isotonic after reconstitution, and the 250 I.U. dosage strength has half the tonicity of the other two dosage strengths after reconstitution.

Another example, Mononine® is a highly purified preparation of Factor IX. Each mL of the reconstituted concentrate contains approximately 100 IU of Factor IX and non-detectable levels of Factors II, VII and X (<0.0025 IU per Factor IX unit using standard coagulation assays). It also contains histidine (approx. 10mM), sodium chlorine (approx. 0.066M) and mannitol (approx. 3%). Hydrochloric acid and/or sodium hydroxide may have been used to adjust pH. Mononine® also contains trace amounts ( = 50 ng mouse protein/100 Factor IX activity units) of the murine monoclonal antibody used in its purification.

For FIX, amino acids, carbohydrates and tensides are specifically preferred formulating agents for pharmaceutical preparations. Therefore, the composition according to the present invention preferably comprises an amino acid, preferably L-histidine or glycine, a carbohydrate, preferably sucrose, a tenside, preferably a polysorbate, especially polysorbate 80, or mixtures thereof as pharmaceutically acceptable carrier(s).

It is also advantageous to provide the composition according to the present invention in lyophilized form due to the significantly increased stability of the lyophilized FIX compared to the FIX in solution. If provided in lyophilized form, the pharmaceutical composition according to the present invention is preferably accompanied with a suitable reconstitution solution containing the carrier including the necessary components for reconstituting the FIX lyophilizate to an administrable pharmaceutical composition.

According to another aspect, the present invention also relates to a method of treating a bleeding disorder, which method comprises the step of administering an effective amount of a pharmaceutical composition according to the present invention to a patient in need thereof. A preferred bleeding disorder to be treated is haemophilia B. The expression "bleeding disorder" in the context of the present invention is of course understood as "bleeding disorders being associated with functional defects of FIX or deficiencies of FIX" and includes bleeding disorders, wherein the cause of the bleeding disorder is due to lack of or reduced function of FIX, e.g. a shortened in vivo half life of FIX, altered binding properties of FIX, genetic defects of FIX, and a reduced plasma concentration of FIX. Genetic defects of FIX comprise for example deletions, additions and/or substitution of bases in the nucleotide sequence encoding FIX whose absence, presence and/or substitution, respectively, has a negative impact on the activity of FIX. Symptoms of such bleeding disorders include, e.g., severe epistaxis, oral mucosal bleeding, hemarthrosis, hematoma, persistent hematuria, gastrointestinal bleeding, retroperitoneal bleeding, tongue/retropharyngeal bleeding, intracranial bleeding, trauma-associated bleeding.

The composition according to the present invention comprising the polymer-coupled FIX is administered by any parenteral (e.g., intravenously, intramuscularly, subcutaneously, or intraperitoneally) or non-parenteral route (e.g., orally).

The present invention further relates to the use of a FIX with a water-soluble hydrophilic polymer covalently coupled to the AP region according to the present invention in the manufacture of a medicament for treating a bleeding disorder. The FIX described herein is used to treat, prevent or alleviate symptoms of the bleeding disorders associated with functional defects of FIX or deficiencies of FIX such as, for example, Hepatitis B. As used herein, a "subject" or a "patient" refers to any warm-blooded animal, such as, for example, a primate, preferably, however, the present invention is used for human patients.

According to another aspect, the present invention relates to a method for the preparation of a blood coagulation factor IX (FIX) with a FIX activation peptide region (AP region), wherein said AP region comprises a covalently coupled water-soluble hydrophilic polymer according to the present invention comprising the following steps:
- providing a FIX molecule comprising a FIX activation peptide region (AP region),
- covalently coupling a mixing a water-soluble hydrophilic polymer to said AP region, and
- isolating a FIX with a covalently coupled water-soluble hydrophilic polymer in said AP region.

It is preferred to use rFIX, especially rhFIX, for the present method; however, also pdFIX may be applied, especially if an immunopurified human pdFIX is used as a starting material for the method according to the present invention. Preferred forms of rhFIX are rhFIX preparations which have been expressed in CHO- or HEK293-derived cells.

In a preferred method, FIX applied resembles the pdFIX as closely as possible; it is therefore preferred, if the rFIX is a rFIX preparation wherein Tyr-155 of FIX is sulfated and/or Ser-158 of FIX is phosphorylated.

In a preferred embodiment of the present invention, the water-soluble hydrophilic polymer is hydroxyethyl starch (HES), polyethylene glycol (PEG), dextran or polysialic acid (PSA).

In a preferred embodiment of the present invention, the water-soluble hydrophilic polymer is attached to FIX via Asn-157 and/or Asn-167 of FIX, especially if the water-soluble hydrophilic polymer is PEG, HES, dextran or PSA, especially HES or PSA.

In an alternatively preferred embodiment the water-soluble hydrophilic polymer is attached to FIX via Ser-158, Thr-159, Thr-163, Thr-169, Ser-171, Thr-172, Ser-174 or Thr-179, especially via Ser-158, Thr-163, Ser-171 or Ser-174, of FIX, especially if the water-soluble hydrophilic polymer is PEG.

It is preferred to use a releasable linker, especially a hydrolysable linker for coupling the water-soluble hydrophilic polymer to FIX.

The present invention can be used to improve FIX medicaments being already on the market by attachment of water-soluble hydrophilic polymers to the FIX molecules in these medicaments. Preferably, rFIX or immunopurified FIX preparation are used for this purpose, such as Benefix® or Mononine®.

The present invention will be further illustrated in the following examples, without any limitation thereto.

### EXAMPLES

### Example 1: Synthesis of human recombinant Factor IX PEG conjugate by modification of carbohydrate residues

For synthesis of rFIX conjugate via carbohydrate residues in the activation peptide a solution of rFIX at a concentration of 1 mg/ml is prepared under conditions described by Van Lenten L and Ashwell G (J.Biol.Chem. 246 (1971), 1889-1894). The buffer used is a 20 mM sodium acetate solution, pH 5.6 and NalO₄ is added to a final concentration of 0.01 M for the oxidation of carbohydrate residues. The oxidation is carried out for 20 min at 4°C, then sodium bisulfite (final concentration 5 mM) is added to stop the reaction. Subsequently mPEG-hydrazide (chain length: 3kD) is added (final concentration 10 mM) and the PEGylation of the rFIX is performed for 1 hr at room temperature. Then the PEGylated rFIX is purified by size-exclusion chromatography. The reaction mixture is applied onto a chromatographic column (size: 26mm x 840mm) filled with Sephacryl S-300 HR (Amersham) and the PEGylated rFIX is separated from the reagents by using 20 mM HEPES - buffer, 150 mM NaCl, pH 7.4 containing 5% trehalose. The modified rFIX is eluted in the void volume as indicated by measurements of rFIX-antigen levels and OD 280nm. The rFIX containing fractions were directly applied to an anion-exchange column (size: 10mm x 108mm) filled with EMD TMAE 650 M (Merck) for further purification. Then the PEGylated rFIX is eluted with 20 mM HEPES buffer, containing 5% trehalose and 1000 mM NaCl. The chemical modification of rFIX used in the present Example 1 involves a periodate oxidation of vicinal hydroxyls of carbohydrates which performed under the conditions described can be rendered relatively selective for sialic acids. Because the rFIX protein used for this experiment is fully sialylated at Asn-157 and Asn-167 while O-linked glycans attached to positions Ser-63. Ser-61, Thr-159, Thr-169, Thr-172 is lacking terminal sialic acid, it could be assumed that modification by PEG is selectively attached to N-linked glycans, which only exist in the activation peptide. This can be confirmed by carbohydrate analysis.

### Example 2: Coupling of dextrane to rFIX

A dextrane (MW 40kD) solution of 6 mg/ml is prepared in 20 mM sodium acetate buffer, pH 6.0 and NalO₄ is added (final concentration 10 mM) to generate free aldehyde groups. The oxidation is carried out for 1 hour at 4°C in the dark, then sodium bisulfite (final concentration 5 mM) is added to stop the reaction. The activated dextrane is dialyzed against 0.1 M sodium phosphate buffer, pH 7.2, containing 0.15 M NaCl (PBS - buffer). Then 2.4 ml of this solution of activated dextrane are added to 10 ml of a solution of recombinant rFIX (concentration: 0.6 mg/ml in PBS buffer). To this mixture 5 ml of a sodium cyanoborhydride solution (64 mg/ml in PBS buffer) are added and incubated at room temperature overnight in the dark. Then 3 ml of a 1.0 M TRIS-HCl solution, pH 7.2, are added to block remaining aldehyde groups and incubated for 1h at room temperature and dialyzed against 20 mM HEPES - buffer, pH 7.4, containing 5% sucrose. Then the dextrane coupled rFIX derivative is further purified by size-exclusion chromatography by applying the mixture onto a chromatography column (size: 50mm x 860mm) filled with Sephacryl S-300 HR (buffer: 20 mM HEPES, 5% sucrose, pH 7.4). The rFIX derivative is eluted in fractions indicated by measurement of rFIX-antigen levels and OD 280nm. These fractions are collected and concentrated by ultrafiltration using a 10 kD regenerated cellulose membrane (Millipore).

### Example 3: Determination of Factor IX activity

Factor IX activity can be measured in the conventional clotting assay using FIX deficient plasma or in an amidolytic assay using a chromogenic substrate. The latter measures FIXa amidolytic activity as for example described by Lenting et al. (J.Biol.Chem. 270 (1995), 14884-14890).

The assay principle of the chromogenic FIX activity is as follows: In presence of thrombin, phospholipids and calcium, first Factor XIa, supplied in the assay at a constant concentration and in excess, activates FIX, present in the tested sample, into FIXa, which forms an enzymatic complex with thrombin activated factor VIII:C, also supplied in the assay at a constant concentration and in excess, phospholipids (PLPs) and Calcium, that activates Factor X, present in the assay system, into Factor Xa. This activity is directly related to the amount of Factor IX, which is the limiting factor. Generated Factor Xa is then exactly measured by its specific activity on Factor Xa chromogenic substrate (SXa-11). Factor Xa cleaves the substrate and releases pNA. The amount of pNA generated is directly proportional to the Factor IXa activity. Finally, there is a direct relationship between the amount of Factor IX in the assayed sample and the Factor Xa activity generated, measured by the amount of pNA released, determined by colour development at 405nm.

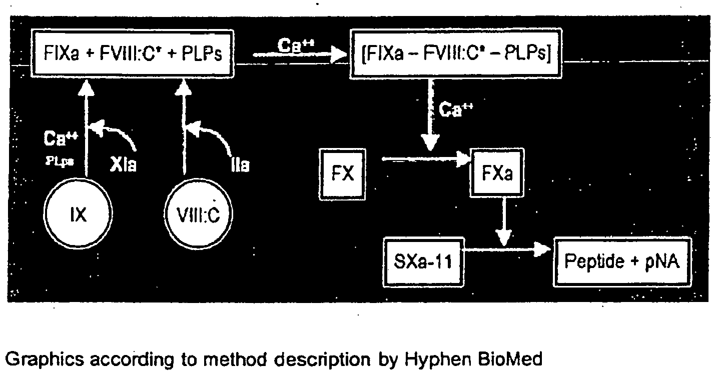

One embodiment of the test is the following: 150 nM of FIXa is incubated in a 96 well plate with chromogenic substrate CH₃SO₂-(D)-CHG-GLY-ARG-pNA in concentrations between 0.5 and 5 mM in 33 % (V/V) ethylene glycol, 100 mM NaCl, 10 mM CaCl₂, 0.2 % (W/V) HSA and 50 mM Tris (ph 7.4). Initial rates of substrate hydrolysis are measured at 37 °C by monitoring absorbance at 405 nm in time. The experimental results are fitted in the Michaelis - Menten equation to obtain km and Kcat values.

The clotting assay for FIX is performed according to the Assay of Human Coagulation Factor IX as described in the current edition of the European Pharmacopoeia and as also described in DIN 58907-1 (Bestimmung der Faktor IX Gerinnungsaktivität (Determination of FIX Clotting Activity) - (FIXC) Teil 1: Referenzmessverfahren für die Einstufenmethode (Part 1: Reference value method for one-step procedure), 2000). The test principle is based on a modified method of measuring partial thromboplastin time. Specificity for FIX is achieved by use of human FIX deficient plasma. In this assay there is a correlation between the FIX activity in a sample and the clotting time. The relationship of the log of FIX activity to the clotting time is linear. The evaluation is performed by the parallel line assay or alternatively by using a reference curve with an appropriate FIX reference preparation.

### Example 4: Pharmacokinetics of modified FIX in hemophilia B animals

Pharmacokinetics and pharmacodynamics of human FIX and FIX variants are preferentially tested in animals with targeted inactivation of the coagulation FIX gene causing hemophilia B. Such a model was for example developed by Kundu RK at al. (Blood. 1998;92:168-74), who replaced a gene segment in the gene encoding for the catalytic domain of the protein. Out of transfected embryonic stem cell clones male chimeric mice were generated and used to found a colony of FIX deficient mice. These mice had a reduced FIX level to approximately 1-2 % of normal mice. This FIX deficiency also translated into a hemorrhagic diathesis resulting in lethal bleeding upon exsanguinations after tail snipping.

Factor IX deficient mice in groups of 10 animals are treated with modified FIX in doses of 1, 10, 30, 100 and 200 units FIX per kg bodyweight equivalent to unmodified FIX. For control purposes additional groups of test animals are treated with unmodified FIX (e.g. human recombinant FIX Benefix®) or formulation buffer at a dose of 10 ml per kg bodyweight. The test substances are applied intravenously and subsets of each treatment group of mice are ex-sanguinated by heart puncture at 5 min, 1 hr, 3 hrs, 6 hrs, 15 hrs, 24 hrs, 48 hrs and 72 hrs after application of the test substances. The blood obtained by heart puncture is tested for FIX activity as described in example 3 and FIX elimination curves are constructed to calculate pharmacokinetic parameters. Human FIX modified by conjugation of PEG by modification of the carbohydrate residues shows a prolonged survival of FIX activity in the blood of hemophilia B knock-out mice. Typical pharmacokinetic improvements are seen by an increase of the area under the curve or the terminal half-life by a factor between 1.2 to 2.5.

### Example 5: Pharmacodynamics of modified FIX in hemophilia B animals

The same animals as described in example 4 are also used for tail tip bleeding experiments to test for the hemostatic potency of FIX and chemically modified FIX. Tail clips of 2 mm are made after treating mice at the same doses as indicated for the pharmacokinetic study. Treatment is performed 5 min before tail clipping and test substances are applied intravenously. Bleeding is performed for 45 min and total blood loss is measured as described by Turecek et al. (Thromb Haemost. 77(1997), 591-599). In addition to measurement of the bleeding intensity and the amount of blood loss also survival is tested in the different treatment groups. Typical blood losses in FIX deficient animals treated with formulation buffer in this experiment are between 200 and 800 µl with a median of around 500 µl while animals treated with human FIX show a reduced blood loss to approximately 200 µl. The same reduced blood loss can be seen in animals treated with modified FIX. Survival after 24 hours in animals treated with recombinant human FIX is doubled to the group of animals treated with formulation buffer. Animals receiving modified FIX show an additional increase in survival by 20 to 50 %.
1. Blood coagulation factor IX (FIX) with a FIX activation peptide region (AP region), wherein said AP region comprises a covalently coupled water-soluble hydrophilic polymer, said polymer being absent from biologically produced FIX.
2. FIX according to item 1, wherein said FIX is recombinant human FIX (rhFIX).
3. FIX according to item 2, wherein said rhFIX is expressed in CHO- or HEK293-derived cells.
4. FIX according to any one of items 1 to 3, wherein Tyr-155 of FIX is sulfated and/or Ser-158 of FIX is phosphorylated.
5. FIX according to any one of items 1 to 4, wherein said water-soluble hydrophilic polymer is hydroxyethyl starch (HES), polyethylene glycol (PEG), dextran or polysialic acid (PSA).
6. FIX according to any one of items 1 to 5, wherein said water-soluble hydrophilic polymer is attached to FIX via Asn-157 and/or Asn-167 of FIX.
7. FIX according to items 6, wherein said water-soluble hydrophilic polymer is HES or PSA.
8. FIX according to any one of items 1 to 7, wherein said water-soluble hydrophilic polymer is attached to FIX via Ser-158, Thr-159, Thr-163, Thr-169, Ser-171, Thr-172, Ser-174 or Thr-179, especially via Ser-158, Thr-163, Ser-171 or Ser-174, of FIX.
9. FIX according to item 8, wherein said water-soluble hydrophilic polymer is PEG.
10. FIX according to any one of items 1 to 9, wherein said water-soluble hydrophilic polymer is attached to FIX via a releasable linker, especially a hydrolysable linker.
11. Pharmaceutical composition containing a FIX according to any one of items 1 to 10 and a pharmaceutically acceptable carrier.
12. The composition according to item 11, wherein FIX has a specific activity of at least 100 international units (IU) of FIX per mg FIX protein, especially at least 200 IU FIX per mg FIX protein.
13. The composition according to item 11 or 12, wherein said pharmaceutically acceptable carrier is an amino acid, preferably L-histidine or glycine, a carbohydrate, preferably sucrose, a tenside, preferably a polysorbate, especially polysorbate 80, or mixtures thereof.
14. The composition according to any one of items 11 to 13, wherein the composition is in lyophilized form.
15. A method of treating a bleeding disorder, the method comprising the step of administering an effective amount of a pharmaceutical composition according to any one of items 11 to 14 to a patient in need thereof.
16. A method according to item 15 wherein said bleeding disorder is haemophilia B.
17. A method for the preparation of a blood coagulation factor IX (FIX) with a FIX activation peptide region (AP region), wherein said AP region comprises a covalently coupled water-soluble hydrophilic polymer according to any one of items 1 to 10 comprising the following steps:
   - providing a FIX molecule comprising a FIX activation peptide region (AP region),
   - covalently coupling a mixing a water-soluble hydrophilic polymer to said AP region, and
   - isolating a FIX with a covalently coupled water-soluble hydrophilic polymer in said AP region.
18. The method according to item 17, wherein said FIX is recombinant human FIX (rhFIX).
19. The method according to item 18, wherein said rhFIX is expressed in CHO- or HEK293-derived cells.
20. The method according to any one of items 17 to 19, wherein Tyr-155 of FIX is sulfated and/or Ser-158 of FIX is phosphorylated.
21. The method according to any one of items 17 to 20, wherein said water-soluble hydrophilic polymer is hydroxyethyl starch (HES), polyethylene glycol (PEG), dextran or polysialic acid (PSA).
22. The method according to any one of items 17 to 21, wherein said water-soluble hydrophilic polymer is attached to FIX via Asn-157 and/or Asn-167 of FIX.
23. The method according to item 22, wherein said water-soluble hydrophilic polymer is HES or PSA.
24. The method according to any one of items 17 to 23, wherein said water-soluble hydrophilic polymer is attached to FIX via Ser-158, Thr-159, Thr-163, Thr-169, Ser-171, Thr-172, Ser-174 or Thr-179, especially via Ser-158, Thr-163, Ser-171 or Ser-174, of FIX.
25. The method according to item 24, wherein said water-soluble hydrophilic polymer is PEG.
26. The method according to any one of items 17 to 25, wherein said water-soluble hydrophilic polymer is attached to FIX via a releasable linker, especially a hydrolysable linker.

## Claims

1. Blood coagulation factor IX (FIX) with a FIX activation peptide region (AP region), wherein said AP region comprises a covalently coupled water-soluble hydrophilic polymer, said polymer being absent from biologically produced FIX.

2. FIX according to claim 1, wherein said FIX is recombinant human FIX (rhFIX).

3. FIX according to any one of claims 1 or 2, wherein Tyr-155 of FIX is sulfated and/or Ser-158 of FIX is phosphorylated.

4. FIX according to any one of claims 1 to 3, wherein said water-soluble hydrophilic polymer is hydroxyethyl starch (HES), polyethylene glycol (PEG), dextran or polysialic acid (PSA).

5. FIX according to any one of claims 1 to 4, wherein said water-soluble hydrophilic polymer is attached to FIX via Asn-157 and/or Asn-167 of FIX.

6. FIX according to any one of claims 1 to 5, wherein said water-soluble hydrophilic polymer is attached to FIX via Ser-158, Thr-159, Thr-163, Thr-169, Ser-171, Thr-172, Ser-174 or Thr-179, especially via Ser-158, Thr-163, Ser-171 or Ser-174, of FIX.

7. FIX according to any one of claims 1 to 6, wherein said water-soluble hydrophilic polymer is attached to FIX via a releasable linker, especially a hydrolysable linker.

8. Pharmaceutical composition containing a FIX according to any one of claims 1 to 7 and a pharmaceutically acceptable carrier.

9. The composition according to claim 8, wherein FIX has a specific activity of at least 100 international units (IU) of FIX per mg FIX protein, especially at least 200 IU FIX per mg FIX protein.

10. A pharmaceutical composition according to claim 9 for use in treating a bleeding disorder.

11. A pharmaceutical composition for use according to claim 10 wherein said bleeding disorder is haemophilia B.

12. A method for the preparation of a blood coagulation factor IX (FIX) with a FIX activation peptide region (AP region), wherein said AP region comprises a covalently coupled water-soluble hydrophilic polymer according to any one of claims 1 to 7 comprising the following steps:
- providing a FIX molecule comprising a FIX activation peptide region (AP region),
- covalently coupling a mixing of water-soluble hydrophilic polymer to said AP region, and
- isolating a FIX with a covalently coupled water-soluble hydrophilic polymer in said AP region.

13. The method according to claim 12, wherein said FIX is recombinant human FIX (rhFIX).

14. The method according to any one of claims 12 or 13, wherein Tyr-155 of FIX is sulfated and/or Ser-158 of FIX is phosphorylated.

15. The method according to any one of claims 12 to 14, wherein said water-soluble hydrophilic polymer is hydroxyethyl starch (HES), polyethylene glycol (PEG), dextran or polysialic acid (PSA).

16. The method according to any one of claims 12 to 15, wherein said water-soluble hydrophilic polymer is attached to FIX via Asn-157 and/or Asn-167 of FIX.

17. The method according to any one of claims 12 to 16, wherein said water-soluble hydrophilic polymer is attached to FIX via Ser-158, Thr-159, Thr-163, Thr-169, Ser-171, Thr-172, Ser-174 or Thr-179, especially via Ser-158, Thr-163, Ser-171 or Ser-174, of FIX.

18. The method according to any one of claims 12 to 17, wherein said water-soluble hydrophilic polymer is attached to FIX via a releasable linker, especially a hydrolysable linker.
